# EUROPÄISCHE PATENTSCHRIFT

(11) **EP 2 418 999 B1**
(45) Veröffentlichungstag und Bekanntmachung des Hinweises auf die Patenterteilung: **28.12.2016**
(21) Anmeldenummer: 10706549.2
(22) Anmeldetag: 02.03.2010
(51) Int. Cl.: A61B 1/05, A61B 1/00

(54) **CHIRURGISCHES INSTRUMENT**
SURGICAL INSTRUMENT
INSTRUMENT CHIRURGICAL

(30) Priorität: 06.03.2009 DE 102009011479
(43) Veröffentlichungstag der Anmeldung: 22.02.2012
(73) Patentinhaber: Olympus Winter&Ibe GmbH, 22045 Hamburg (DE)
(72) Erfinder: WIETERS, Martin, 22885 Barsbüttel (DE); SCHÖLER, Uwe, 22955 Hoisdorf (DE)
(74) Vertreter: Seemann & Partner Patentanwälte mbB
(86) Internationale Anmeldenummer: PCT/EP2010/001277
(87) Internationale Veröffentlichungsnummer: WO 2010/099927

(56) Entgegenhaltungen:
- EP-A1- 1 455 216
- JP-A- 2004 261 362
- US-A- 6 080 101
- US-A1- 2003 169 333
- US-A1- 2008 086 033
- US-B1- 6 547 722

## Beschreibung

Die Erfindung betrifft ein chirurgisches Instrument mit einem hermetischen Raum, wobei wenigstens eine elektrische Verbindung vom hermetischen Raum nach außerhalb des hermetischen Raums zu einem Außenraum vorgesehen ist.

Ein derartiges chirurgisches Instrument ist beispielsweise aus DE 10 2006 015 176 B3 bekannt und ist beispielsweise auch in Fig. 1 dieser Patentanmeldung gezeigt.

Dort ist ein starres medizinisches Videoendoskop mit einem Systemrohr gezeigt, das distal mit einem Fenster versehen ist und das in seinem distalen Endbereich ein Objektiv und eine Videokamera aufnimmt, die mit elektrischen Leitungen durch eine proximale Öffnung des Systemrohres nach außen angeschlossen ist, wobei die Leitungen in der Öffnung einen Verschluss aus Vergussmasse durchlaufen.

US 6 080 101 A1 offenbart eine TV-Kamera, die in einem Endoskop angeordnet ist, wobei zur Versorgung der TV-Kamera elektrische Leitungen über eine Art Steckerkontakt mit der TV-Kamera verbunden sind.

US 2003/0169333 A1 offenbart ein Endoskop, bei dem Videokomponenten über elektrische Leitungen und einen Stecker nach außen hin verbunden sind.

EP 1 455 216 A1 offenbart ein Videoendoskop, bei dem mittels Steckverbindung eine elektrische Verbindung von innerhalb nach außerhalb des hermetischen Raums ermöglicht ist.

US 2008/0086033 A1 offenbart ein autoklavierbares medizinisches Instrument. Es ist ein Gehäuse eines Videoendoskops gezeigt.

Es ist Aufgabe der vorliegenden Erfindung, eine alternative Lösung anzubieten, ein chirurgisches Instrument mit einem hermetischen Raum vorzusehen, wobei insbesondere die elektrische Leitungsdurchführung hermetisch ausgestaltet sein soll.

Gelöst wird diese Aufgabe durch ein chirurgisches Instrument mit einem hermetischen Raum, wobei wenigstens eine elektrische Verbindung von dem hermetischen Raum nach außerhalb des hermetischen Raums zu einem Außenraum vorgesehen ist, wobei die wenigstens eine elektrische Verbindung auf einem elektrisch isolierenden Substrat aufgebracht ist, wobei wenigstens auf der elektrischen Verbindung eine erste Isolationsschicht aufgebracht ist und auf der ersten Isolationsschicht eine weitere geeignete Schicht aufgebracht ist, die für eine hermetische Verbindung geeignet ist und die weiterhin mit einer Wandung des chirurgischen Instruments hermetisch verbunden ist.

Die elektrische Verbindung kann hierbei beispielsweise eine Leiterbahn oder eine Leitung sein. Damit wird der hermetische Raum in dem chirurgischen Instrument wenigstens durch eine auf dem elektrisch isolierenden Substrat aufgebrachte Multischicht in Verbindung mit einer Wandung, die beispielsweise mit einem Außenrohr des chirurgischen Instruments hermetisch verbunden ist, hergestellt. Es wird somit eine Durchführungsvorrichtung umfassend ein Substrat, wenigstens eine elektrische Verbindung und eine Isolationsschicht und wenigstens eine Metallschicht gebildet. Diese Schichten sind hierbei fest miteinander, insbesondere über chemische Verbindungen, verbunden. Die Metallschicht dient dann als die für die hermetische Verbindung geeignete Schicht. Die elektrische Verbindung ist auch fest mit dem Substrat und der Isolationsschicht, insbesondere über chemische Verbindungen, verbunden.

Das chirurgische Instrument weist somit eine Durchführungsvorrichtung auf, die hermetisch in einem Rohr des chirurgischen Instruments mit dem Rohr verbunden ist.

Die andere Seite des hermetischen Raums beim chirurgischen Instrument kann beispielsweise wie in DE 10 2006 015 176 B3 im Bereich eines Fensters sein, wo eine hermetische Versiegelung vorgesehen ist. Dieses kann auch wie in DE 196 47 855 B4 im Bereich eines Kristallfilters sein. Die in dieser Erfindung beschriebene hermetische Verbindung betrifft insofern vorzugsweise den proximalen Bereich des chirurgischen Instruments. Sie könnte allerdings auch am distalen Ende des chirurgischen Instruments angewendet werden.

Vorzugsweise ist die hermetische Verbindung eine Lötung. Die elektrische Verbindung, die auf dem elektrisch isolierenden Substrat aufgebracht ist, kann eine elektrisch leitfähige Beschichtung, beispielsweise ein Metall, sein. Die verschiedenen Schichten bzw. auch die elektrische Verbindung, die entsprechend gemäß der Erfindung aufgebracht werden, können mittels chemischer Dampfauftragung (CVD Chemical Vapour Deposition), Elektronensputtern, lonensputtern, Laserablation oder ähnlichen Methoden aufgebracht werden. Die für die hermetische Verbindung geeignete Schicht ist bevorzugt aus einem Metall, insbesondere Gold oder Silber.

Vorzugsweise ist der hermetische Raum in einem Rohr, insbesondere eines Endoskops, insbesondere eines Laparoskops, vorgesehen. Das Rohr kann zylinderförmig sein oder auch eine andere Form aufweisen.

Durch die Erfindung können, vom Durchmesser her, sehr kleine Rohre Verwendung finden, da die hermetische Durchführung von elektrischen Leitungen bzw. elektrischen Verbindungen sehr klein ausgestaltet werden können. Es kann insbesondere auf großbauende hermetische Steckkontakte, wie in US 7 410 462 B2 oder in DE 196 47 855 B4 gezeigt ist, verzichtet werden.

Vorzugsweise weist das elektrisch isolierende Substrat eine Wärmeleitfähigkeit von mehr als 1 W/mK, insbesondere mehr als 10 W/mK, insbesondere größer 200 W/mK auf. Vorzugsweise ist eine entsprechend hohe Wärmeleitfähigkeit vorgesehen, um so eine Wärmeabfuhr, beispielsweise von der Wärme, die durch Lampen in dem hermetischen Raum erzeugt wird, zu ermöglichen. Als Materialien hierfür eignet sich beispielsweise eine Keramik wie Bornitrid, die eine Wärmeleitfähigkeit von bis zu ca. 400 W/mK aufweist, Aluminiumnitrid (180 W/mK bis 200 W/mK), Siliziumcarbid (60 W/mK bis 160 W/mK), Aluminiumoxid (20 W/mK bis 50 W/mK), Siliziumoxid (1 W/mK bis 10 W/mK), Siliziumnitrid (ungefähr 30 W/mk bis 180 W/mk).

Vorzugsweise ist das elektrisch isolierende Substrat wenigstens ein Teil einer flexiblen Leiterplatte, wobei insbesondere die wenigstens eine elektrische Verbindung wenigstens ein Leiter der Leiterplatte ist.

Durch diese bevorzugte Ausführungsform sind weitere Verbindungen von Leitern, Kabeln oder flexiblen Leiterplatten zu den elektrischen Vorrichtungen in dem hermetischen Raum zu vermeiden, da diese selbst die elektrische Verbindung darstellt. Die flexible Leiterplatte ist dann mit den entsprechenden Schichten versehen, um so eine hermetische Verbindung zu ermöglichen.

Vorzugsweise sind mehrere elektrische Verbindungen vorgesehen, die insbesondere symmetrisch um das Substrat angeordnet sind. Durch diese bevorzugte Maßnahme können große Datenmengen schnell weitergegeben werden und auch entsprechend mehrere eine elektrische Versorgung benötigende Vorrichtungen im chirurgischen Instrument mit Strom versorgt werden.

Vorzugsweise erstreckt sich von dem Rohr im Bereich des elektrisch isolierenden Substrats eine Wandung, insbesondere radial, nach innen, wobei die Wandung, insbesondere radial, das elektrisch isolierende Substrat umgibt und mit der für die hermetische Verbindung geeigneten Schicht umlaufend hermetisch verbunden ist.

Im Rahmen der Erfindung bedeutet radial nicht notwendigerweise nur eine kreisförmige Erstreckung nach innen. Es können auch Rohre vorgesehen sein, die nicht im Querschnitt kreisförmig sind sondern anders geformt sind, beispielsweise elliptisch oder quadratisch bzw. rechteckig. Es können auch elliptische oder polygonförmige Rohre vorgesehen sein. Insbesondere kann das elektrisch isolierende Substrat auch im Querschnitt eckig ausgebildet sein, wobei die hermetische Verbindung auch umlaufend hermetisch ausgebildet ist, wobei im Rahmen der Erfindung der Begriff radial sich auf diverse geometrische Formen bezieht.

Vorzugsweise umfasst die Wandung wenigstens zwei Schalenelemente, die insbesondere miteinander hermetisch gefügt sind.

Die Aufgabe wird ferner durch ein chirurgisches Instrument mit einem hermetischen Raum gelöst, wobei eine Mehrzahl von elektrischen Verbindungen von dem hermetischen Raum nach außerhalb des hermetischen Raums zu einem Außenraum vorgesehen ist, das vorzugsweise die vorstehenden erfindungsgemäßen bzw. bevorzugten Merkmale aufweist und das dadurch weitergebildet ist, dass die Mehrzahl von elektrischen Verbindungen auf einer Oberfläche eines elektrisch isolierendem Körpers aufgebracht sind, wobei der Körper eine Längserstreckung von dem hermetischen Raum nach außerhalb des hermetischen Raums aufweist, wobei der Körper hermetisch mit einem Rohr verbunden ist, wobei die Oberfläche des Körpers mit der Mehrzahl von elektrischen Verbindungen bedruckt ist und/oder die elektrischen Verbindungen in Form einer Metallisierung auf die Oberfläche des Körpers aufgebracht sind.

Das Rohr ist vorzugsweise ein Metallrohr. Durch Vorsehen der Mehrzahl von elektrischen Verbindungen auf einer Oberfläche eines elektrisch isolierenden Körpers, ist es auf einfache Weise möglich, eine Vielzahl von elektrischen Verbindungen an einem Anschlussstück vorzusehen, so dass auf sichere Weise die elektrische Verbindung von elektronischen Komponenten beispielsweise in einem Videoendoskop von einem hermetisch dichten Raum nach außerhalb des hermetisch dichten Raums ermöglicht ist. Bei Videoendoskopen wird die Anzahl der elektrischen Zuleitungen mit der Entwicklung der Videokameras immer größer. Beispielsweise werden derzeit bis 39 Steckerpins bzw. Steckerstifte verwendet. Hiermit wir der Aufbau von hermetisch dichten Steckern beispielsweise in einem Glasverguss oder einer anderen hermetischen Dichtung immer schwieriger. Die Steckerpins bzw. Steckerstifte werden hierbei immer dünner und empfindlicher.

Durch das vorgeschlagene chirurgische Instrument bzw. die vorgeschlagene Durchführungsvorrichtung, umfassend den elektrisch isolierenden Körper mit der Mehrzahl von elektrischen Verbindungen, die auf einer Oberfläche des elektrisch isolierenden Körpers aufgebracht sind, ist es möglich, anstelle der Steckerstifte entsprechende Leiterbahnen zu verwenden. Bei dem isolierenden Körper kann es sich beispielsweise um einen Keramikstift mit einer Längserstreckung handeln, entlang der auch die elektrischen Verbindungen vorgesehen sind. Hierdurch können sehr kleine bzw. schmale elektrische Verbindungen vorgesehen werden. Vorzugsweise wird der elektrisch isolierende Körper bzw. Keramikstift mit den elektrischen Leitern bzw. elektrischen Verbindungen bedruckt. Die hierdurch entstehende robuste elektrische Verbindung kann dann beispielsweise mit einem Glasverguss oder anderen Vergussarten hermetisch dicht vergossen werden.

Vorzugsweise wird eine weitere Schicht zumindest auf einen Teil der elektrischen Verbindungen aufgebracht, beispielsweise eine weitere Keramikschicht oder eine andere isolierende Schicht und auf diese Schicht eine metallische Schicht, die für eine hermetische Verbindung geeignet ist. An diese metallische Schicht kann dann eine hermetische Verbindung zu einem Rohr, beispielsweise dem Außenrohr des chirurgischen Instruments, beispielsweise durch Anlöten an eine von dem Außenrohr des chirurgischen Instruments sich nach innen erstreckende Fläche vorgenommen werden.

Vorzugsweise weist der Körper längsaxial sich erstreckende Nuten auf, wobei wenigstens ein Teil der Mehrzahl der elektrischen Verbindungen in den Nuten angeordnet ist. Hierdurch kann eine sehr große Anzahl von elektrischen Zuleitungen bzw. Verbindungen vorgesehen werden, die sich gegenseitig nicht stören. Insbesondere können so auf wenigstens zwei Ebenen bzw. unterschiedlichen Lagen elektrische Verbindungen vorgesehen werden.

Bei einem zylinderförmigen isolierenden Körper, beispielsweise einem entsprechenden Keramikstift, sind dann vorzugsweise elektrische Verbindungen auf einem Durchmesser im Schnitt des isolierenden Körpers angeordnet, der beispielsweise kleiner ist als der Außendurchmesser des isolierenden Körpers. Hierdurch sind die elektrischen Verbindungen sehr gut vor Beschädigungen geschützt.

Zudem kann quasi die doppelte Anzahl von elektrischen Verbindungen vorzugsweise dadurch erreicht werden, dass auf einer anderen Ebene, nämlich beispielsweise der äußeren Oberfläche auf dem Außendurchmesser des isolierenden Körpers, weitere elektrische Verbindungen aufgebracht werden. Vorzugsweise weisen die Nuten einen rechteckigen oder V-förmigen Querschnitt auf.

Wenn vorzugsweise im Fall der V-förmigen Nuten jeweils eine elektrische Verbindung auf eine Flanke der jeweiligen Nut aufgebracht ist, ist eine relativ einfache Herstellung der elektrischen Verbindungen möglich. Diese können dann nämlich durch Aufdrucken oder Aufsputtern eines Metalls von einer Seite während einer Drehung oder mehrerer langsamer Drehungen des elektrisch isolierenden Körpers vorgenommen werden. Die elektrische Verbindung kann hierbei eine Flanke vollständig ausfüllen oder auch nur einen Teil bedecken. Vorzugsweise endet die elektrische Verbindung mit einem Abstand zur äußeren Oberfläche des isolierenden Körpers in der jeweiligen Nut. Vorzugsweise ist ein Teil der Mehrzahl der elektrischen Verbindungen außerhalb der Nuten vorgesehen.

Im Rahmen der Erfindung beinhaltet der Begriff eines rechteckförmigen Ausschnitts einer Nut auch einen U-förmigen Querschnitt.

Ferner ist vorzugsweise das Rohr das Innen- oder Außenrohr des chirurgischen Instruments oder mit dem Innen- oder Außenrohr des chirurgischen Instruments hermetisch abgeschlossen. Das Innen- oder Außenrohr ist dabei insbesondere ein hermetisch dichtes Rohr.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: einen schematischen Längsschnitt durch ein Systemrohr eines Videoendoskops gemäß dem Stand der Technik,
- Fig. 2: einen schematischen Teil eines Längsschnitts durch ein erfindungsgemäßes chirurgisches Instrument,
- Fig. 3: ein schematisches radiales Schnittbild der Ausführungsform gemäß Fig. 2,
- Fig. 4: ein schematisches axiales Schnittbild eines Teils von Fig. 2,
- Fig. 5: ein schematisches radiales Schnittbild einer weiteren erfindungsgemäßen Ausführungsform eines entsprechenden elektrisch isolierenden Substrats mit weiteren Schichten,
- Fig. 6: ein schematisches radiales Schnittbild eines Teils einer erfindungsgemäßen Ausführungsform,
- Fig. 7: eine schematische Schnittdarstellung entlang A-A gemäß Fig. 6,
- Fig. 8: eine schematische radiale Schnittdarstellung durch den Innenbereich eines chirurgischen Instruments,
- Fig. 9: eine schematische axiale Schnittdarstellung entlang B-B aus Fig. 8,
- Fig. 10: einen schematischen Teil eines Längsschnitts durch einen Teil eines erfindungsgemäßen chirurgischen Instruments,
- Fig. 11: eine schematische dreidimensionale Darstellung eines Teils der Fig. 10 in einer alternativen Ausführungsform,
- Fig. 12: eine schematische Seitenansicht eines Teils des Körpers aus Fig. 11 in einer anderen Ausführungsform und
- Fig. 13: eine schematische Seitenansicht eines Teils einer anderen erfindungsgemäßen Ausführungsform.

In den folgenden Figuren sind jeweils gleiche oder gleichartige Elemente bzw. entsprechende Teile mit denselben Bezugsziffern versehen, so dass von einer entsprechenden erneuten Vorstellung abgesehen wird.

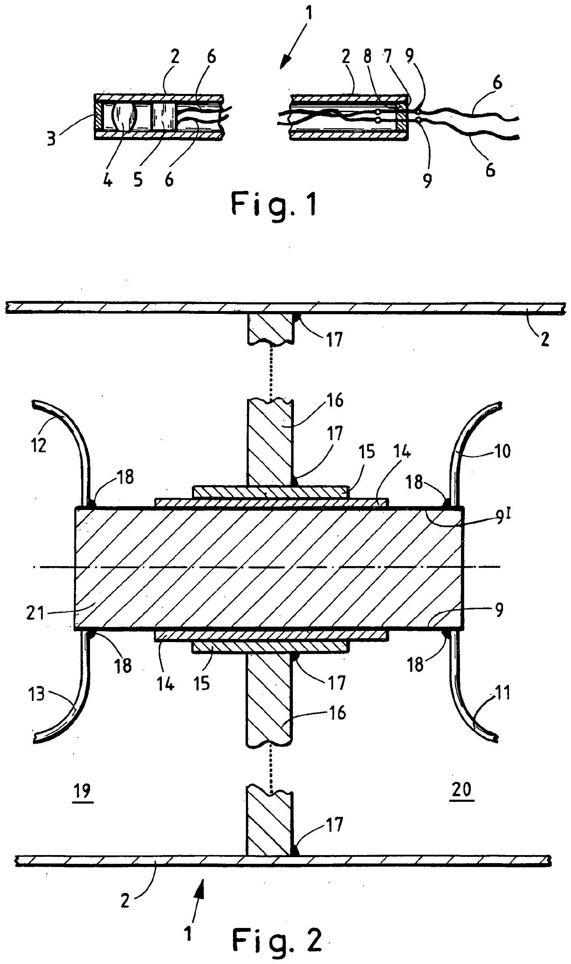

Fig. 1 zeigt schematisch einen Längsschnitt durch ein Systemrohr eines Videoendoskops gemäß dem Stand der Technik aus DE 10 2006 015 176 B3.

Fig. 1 zeigt ein starres medizinisches Videoendoskop 1 mit einem aus Metall bestehenden Systemrohr 2, das am distalen Ende mit einem eingelöteten Fenster 3 verschlossen ist, hinter dem im distalen Endbereich des Systemrohres 2 ein schematisch dargestelltes Objektiv 4 und eine Videokamera 5 angeordnet sind, das über die Leitungen nach außen angeschlossen ist. Im vereinfacht dargestellten Beispiel der Fig.1 sind dies zwei elektrische Leitungen 6.

Die elektrischen Leitungen 6 verlaufen durch die proximale Öffnung 7 des Systemrohres 2, die mit einer Vergussmasse 8 verschlossen ist. Die elektrischen Leitungen 6 sind in diesem Bereich als nicht isolierte Durchführungsleiter 9 ausgebildet. Nach dem üblichen Stand der Technik ist zur ausreichenden hermetischen Abdichtung des Innenraumes des Systemrohrs 2 als Vergussmasse 8 Glas vorgesehen, das flüssig in das Systemrohr 2 und um die Durchführungsleitern 9 eingeschmolzen wird. Eine einfache Ausbildung der Vergussmasse 8 aus einem Kunststoff kann zu einer unzureichenden Gasdichtigkeit führen.

Fig. 2 zeigt schematisch einen Teil eines erfindungsgemäßen chirurgischen Instruments 1, beispielsweise ein Videoendoskop, ein Laparoskop, ein Endoskop oder ein ähnliches Instrument in einer ersten erfindungsgemäßen Ausführungsform, wobei der Teil gezeigt ist, der für eine hermetische Versiegelung eines hermetischen Raums 19 von einem Außenraum 20 dargestellt ist.

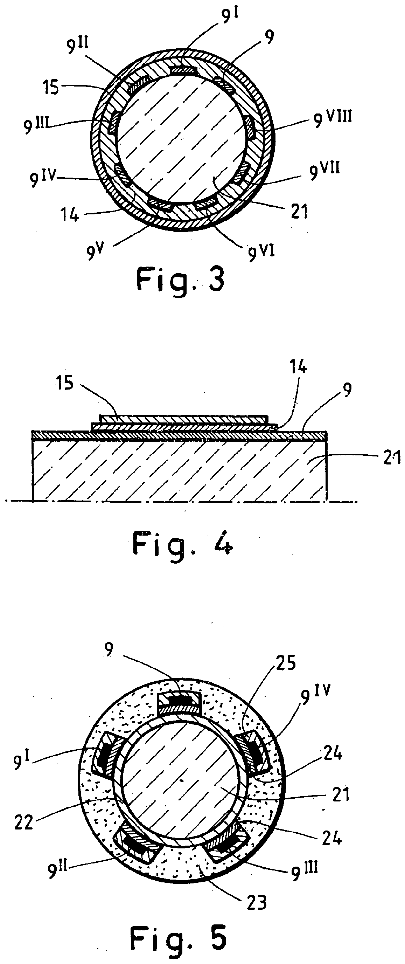

In diesem Ausführungsbeispiel ist das Systemrohr 2 zylinderförmig ausgebildet. Es kann allerdings auch andere Formen aufweisen. Zur hermetischen Durchkontaktierung bzw. zur hermetischen elektrischen Verbindung wird auf der Außenseite eines zylinderförmigen Substrates 21, das bevorzugterweise ein nicht elektrisch leitendes, aber Wärme leitendes Material aufweist, eine Leiterbahnstruktur in Form von Durchführungsleitern 9, 9^{I} und weiteren Durchführungsleitern, die dann in Fig. 3 dargestellt sind, aufgebracht.

Das Aufbringen der Leiterbahnstruktur kann mittels Drucken, CVD, Magnetsputtern, Laserablation und ähnlichem geschehen.

Es wird dann eine Isolationsschicht 14 aufgebracht und auf der Isolationsschicht eine Metallschicht 15. An die Metallschicht 15 grenzt die Wandung 16, die zum Systemrohr 2 führt. Die Wandung ist mit dem Systemrohr 2 und der Metallschicht 15 hermetisch verlötet.

Hierdurch wird eine hermetische Versiegelung des hermetischen Raums 19 ermöglicht.

Zur Durchkontaktierung bzw. zur Verbindung mit den entsprechenden Leitungen sind flexible Leiterplatten 10, 11, 12, 13 vorgesehen, die mit einem entsprechenden Lot 18 auch mit der jeweiligen Leiterbahn 9, 9^{I} und auch den weiteren Leiterbahnen aus Fig. 3 elektrisch verbunden sind. Es ist zu erkennen, dass an den Endflächen des Substrats die Leiterbahnen bzw. die Durchführungsleiter 9, 9^{I} und auch die weiteren Durchführungsleitern 9^{II}-9^{VIII}, die in Fig. 3 dargestellt sind, freiliegen. Die Isolationsschicht 14 und die Metallschicht 15 sind vollständig um das Substrat 21 herum angeordnet. Das Substrat kann-eine Keramik sein, die vorstehend genannt ist.

Zur Stabilisierung der flexiblen Leiterplatten 10, 11 am proximalen Ende des chirurgischen Instruments bzw. Videoendoskops 1 können diese zum Beispiel mit Kunststoff vergossen sein. Hierdurch können die Lotstellen 18 bzw. die flexiblen Leiterplatten 10, 11 vor Zugbelastung geschützt werden.

Fig. 3 zeigt schematisch einen Querschnitt durch die Durchführungsvorrichtung mit dem Substrat 21 gem. Fig. 2. Die Durchführungsleiter 9-9^{VIII} können aufgedampft und strukturiert sein. Entsprechend können die Isolationsschicht 14 und die Metallschicht 15 auch aufgedampft sein. Es ist zu erkennen, dass das Substrat 21 im Schnitt rund ist. Symmetrisch um das Substrat 21 sind die entsprechenden Durchführungsleiter 9-9^{VIII} angeordnet. Um diese herum ist die Isolationsschicht 14 angeordnet und darum herum die Metallschicht 15. Die Isolationsschicht 14 kann ein Siliziumoxid oder ein Siliziumnitrid oder eine andere Keramik sein. Die Durchführungsleiter 9-9^{VIII} können aus Gold, Kupfer oder Silber sein. Die äußere Metallschicht, die auch als Lötschicht bezeichnet werden kann, kann beispielsweise aus Gold sein.

In Fig. 4 ist noch eine Schnittdarstellung eines Teils der Ausführungsform gemäß Fig. 3, der besseren Veranschaulichung wegen ohne die Wandung 16, dargestellt.

Fig. 2 bis 4 zeigen eine Ausführungsform ohne Schirmung. Dahingegen ist in Fig. 5 eine schematische Schnittdarstellung einer Ausführungsform mit Schirmung dargestellt. Es ist zu erkennen, dass zunächst um das Substrat 21 eine Zwischenschicht 22 vorgesehen ist, die eine Metallschicht sein kann und zusammen mit der Löt- und Abschirmungsschicht 23 zur Abschirmung dient. Die Leiterbahnen bzw. Durchführungsleiter 9-9^{VIII} sind auf einer Isolationsschicht 24 angeordnet. Um die Durchführungsleiter 9-9^{IV} ist eine zweite Isolationsschicht 25 angeordnet. Die entsprechende Schichtung und die Durchführungsleiter können auch entsprechend aufgedampft sein. Die Isolationsschichten 24 und 25 können aus Siliziumnitrid, Siliziumoxid oder einer anderen Keramik sein.

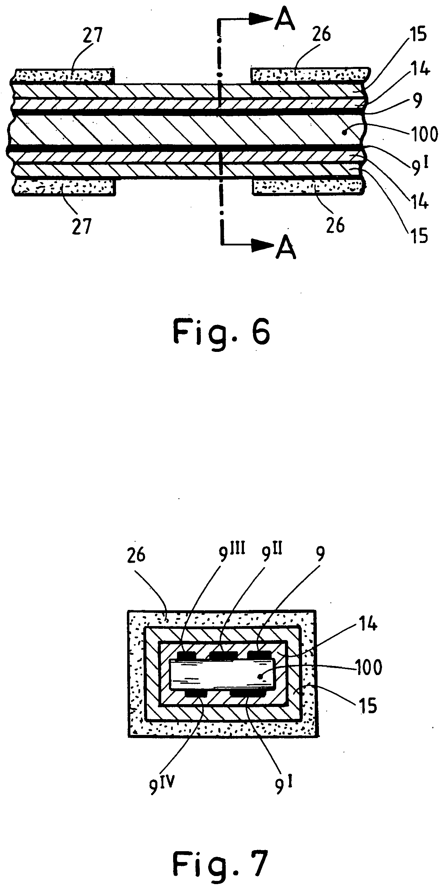

Fig. 6 zeigt eine schematische Darstellung einer erfindungsgemäßen Durchführungsvorrichtung in einer längsaxialen Schnittdarstellung. Es ist eine quaderförmige flexible Leiterplatte 100 als Substrat vorgesehen, die auch aus einem Kunststoff sein kann.

Fig. 7 zeigt eine schematische Schnittdarstellung entlang A-A aus Fig. 6. Um die flexible Leiterplatte 100 sind fünf Durchführungsleiter 9-9^{IV} aufgebracht, wovon drei oberhalb der Leiterplatte 100 und zwei unterhalb der Leiterplatte 100 aufgebracht sind. Die Durchführungsleiter 9-9^{IV} können auch integraler Bestandteil der Leiterplatte 100 sein. Es können auch an den Seiten Durchführungsleiter angebracht sein oder eine andere Anzahl.

Um die Durchführungsleiter und die flexible Leiterplatte 100 ist eine Isolationsschicht 14 vorgesehen. Um die Isolationsschicht 14 ist eine Metallschicht 15 vorgesehen. Die Isolationsschicht 14 und die Metallschicht 15 umgibt das Substrat mit den Durchführungsleitern 9-9^{IV} im Schnitt hier vollständig.

An den Rändern, also in Fig. 6 links und rechts angedeutet, sind zwei weitere Isolationsschichten 26 und 27 um die Metallschicht 25 bzw. auf der Metallschicht 25 aufgebracht. Diese Schichten sind, was in Fig. 6 gezeigt ist, im mittleren Bereich nicht aufgebracht und auch an den Endbereichen, was in Fig. 6 nicht dargestellt ist, auch nicht aufgebracht, um eine entsprechende Verbindung der Durchführungsleiter 9 bis 9^{IV} zu ermöglichen.

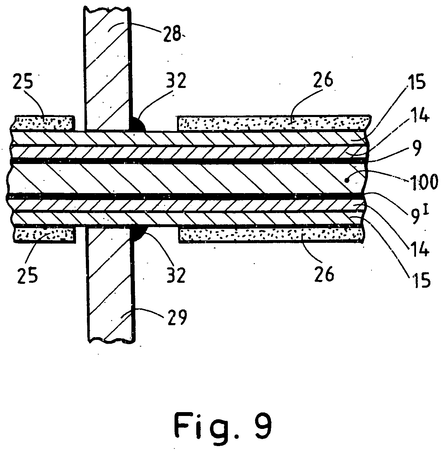

Eine Verbindung mit einer entsprechenden ersten Halbschale 28 und der entsprechenden zweiten Halbschale 29 zu dem nicht dargestellten Systemrohr 2 ist in Fig. 9 dargestellt. Dort ist auch ein hermetisches Lot 32 dargestellt.

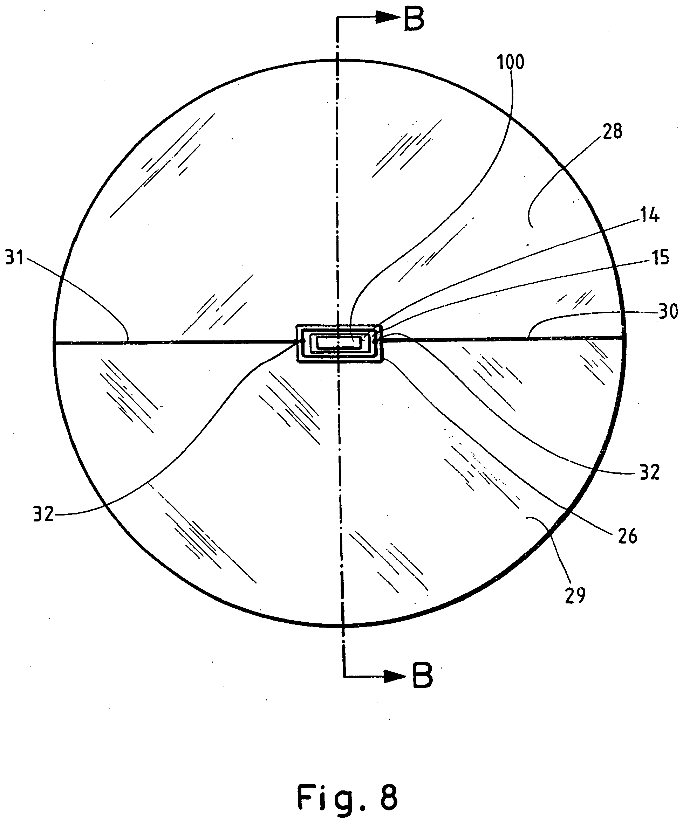

In Fig. 8 ist eine Struktur dargestellt, die in ein nicht dargestelltes Systemrohr 2 eingebracht wird, um eine hermetische Versiegelung vorzusehen. Es sind eine erste Halbschale 28 und eine zweite Halbschale 29 vorgesehen, die an den Stoßstellen 30 und 31 hermetisch gefügt sind. Am äußeren Rand der Halbschalen 28 und 29 wird dann das Lot angebracht, das eine Verbindung mit dem nicht dargestellten Systemrohr 2 ermöglicht. Auch hierbei handelt es sich um eine hermetische Verbindung. Die hermetische Verbindung mit der Durchführungsvorrichtung umfassend die flexible Leiterplatte 100, die in Fig. 8 der besseren Darstellbarkeit wegen nicht dargestellten Durchführungsleitungen, der Isolationsschicht 14 und der Metallschicht 15, an der das Lot 32 angebracht ist, ist auch in Fig. 8 dargestellt. Es ist auch noch schematisch die Isolationsschicht 26 dargestellt. Diese steht über die durch die Halbschalen 28, 29 sich ergebende Öffnung für die Durchführungsvorrichtung hinaus.

Durch die Ausführungsformen gemäß den Figuren 6 bis 9 ist eine besonders einfache Durchführung von Leitern möglich, da direkt eine flexible Leiterplatte, die mit entsprechenden elektrischen Komponenten im hermetischen Raum verbunden werden, durchgeführt wird. Auf die flexible Leiterplatte 100 werden die entsprechenden weiteren Schichten auch aufgebracht, z.B. aufgedampft. Durch die aufgebrachte Isolationsschicht 26 und 27 lässt sich eine eventuelle Kriechstrecke zwischen den aufgebrachten Schichten der flexiblen Leiterplatte 100 verlängern. Hierdurch kann eine elektrische Durchkontaktierung direkt und ohne weitere Lötstellen oder Steckverbindungen erfolgen. Trotzdem ist eine hermetische Lötung vorhanden. Die Maße, die bei der Durchführungsvorrichtung erfindungsgemäß verwendet werden, sind deutlich kleiner als bei herkömmlichen Lösungen mit hermetischen Steckern. Hierdurch können auch deutlich schlankere Rohre Verwendung finden.

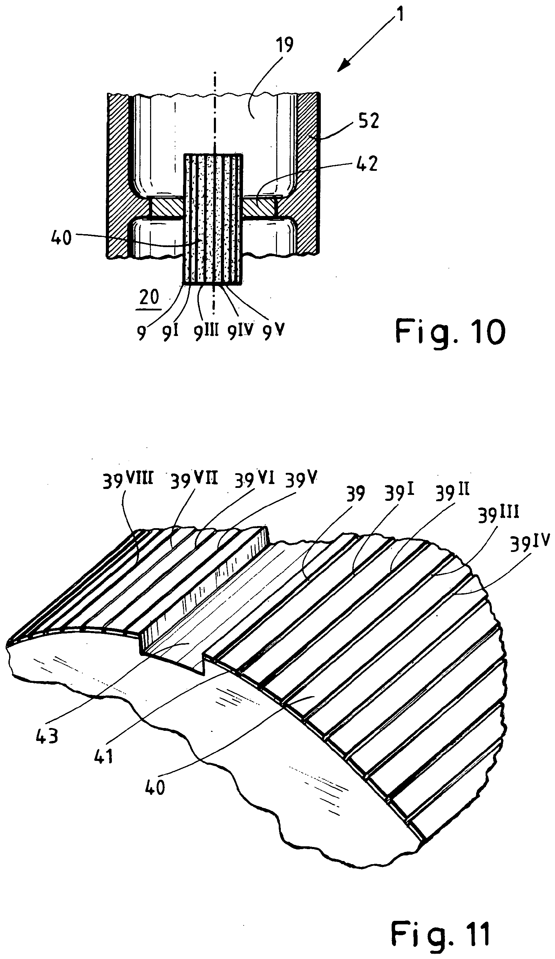

Fig. 10 zeigt einen schematischen Teil eines Längsschnitts durch ein erfindungsgemäßes chirurgisches Instrument in einer weiteren Ausführungsform. Auch hier ist ein hermetisch abgedichteter Raum 19 vorgesehen und ein nicht hermetischer Raum 20. Zur Verbindung dieser beiden Räume und zur Durchführung von elektrischen Leitungen ist ein Isolierkörper 40 vorgesehen, auf dem elektrische Leitungen in Form von elektrisch leitenden Schichten aufgebracht, beispielsweise gedruckt, sind. Der Isolierkörper 40 ist über einen Glasverguss 42 mit dem Rohr 52 verbunden. Hierdurch wird eine hermetische Verbindung erzeugt. Das Rohr 52 kann das Außenrohr des chirurgischen Instruments 1 sein oder aber mit dem Außenrohr des chirurgischen Instruments, insbesondere hermetisch, verbunden sein. Die Leiterbahnen bzw. elektrischen Verbindungen 9-9^{VIII} können, beispielsweise wie in Fig. 10 angedeutet, auf der Oberfläche des Isolierkörpers 40 aufgedruckt oder aufgedampft sein. Hierbei kann es sich jeweils um Metallschichten handeln, die in Längserstreckung des Isolierkörpers angeordnet sind.

Fig. 11 zeigt eine schematische dreidimensionale Darstellung einer anderen Ausführungsform des Isolierkörpers 40. Es sind entsprechende Nuten 41 in die Oberfläche des Isolierkörpers 40 eingebracht. Auf dem Boden der Nuten 40 sind entsprechende elektrische Verbindungen in Form jeweils eines Durchführungsleiters 39-39^{VIII} vorgesehen. Für eine eindeutige Zuordnung der entsprechenden elektrischen Verbindungen mit einem Anschlussstecker ist eine Passnut 43 vorgesehen. Alternativ hierzu kann auch ein exzentrisches Sackloch vorgesehen sein, das mit einem entsprechend passenden Stift eines auf den Isolierkörper 40 anbringbaren Steckers in Verbindung gebracht werden kann.

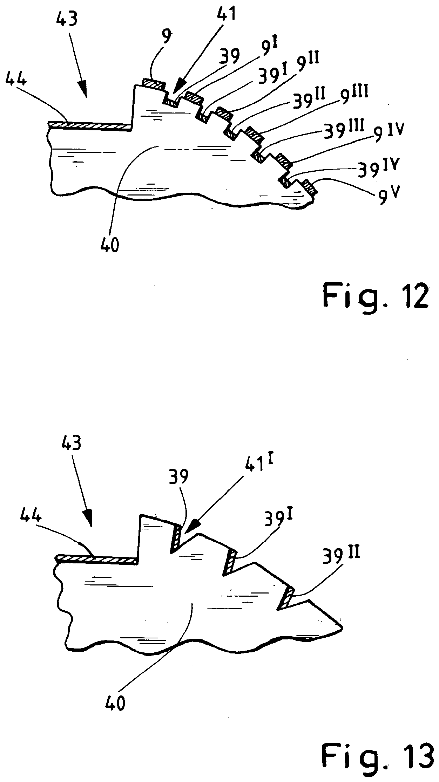

Fig. 12 zeigt einen Ausschnitt einer Seitenansicht des Isolierkörpers 40, in einer weiteren Ausführungsform, wobei rechteckförmige Nuten entlang der Längsachse des Isolierkörpers 40 vorgesehen sind. Die Nuten sind mit der Bezugsziffer 41 gekennzeichnet. In den Nuten sind entsprechende Durchführungsleiter 39 bis 39^{IV} vorgesehen. Auf einer anderen Ebene, nämlich auf der äußeren Oberfläche des Isolierkörpers 40, sind weitere Durchführungsleiter 9-9^{V} angeordnet. Hierdurch können bei einem Umfang von beispielsweise 31,4 mm, was einem Durchmesser von 10 mm des Isolierkörpers 40 entspricht, mehr als 400 Leiterbahnen mit einer Breite von 75 µm untergebracht werden. Durch die Verwendung von kleinen rechteckförmigen Längsnuten über den Umfang verteilt, wird eine zweite Ebene von Leiterbahnen geschaffen.

Fig. 13 zeigt eine andere Ausführungsform des Isolierkörpers 40 in einer schematischen Seitenansicht. Es sind V-förmige Nuten längsaxial in den Isolierkörper 40 eingebracht. An der jeweiligen linken Flanke der jeweiligen Nut 41' sind die Durchführungsleiter 39 bis 39^{VIII} aufgebracht. Es ist vorgesehen, dass in jeder Nut ein Durchführungsleiter angeordnet ist. Auch hier ist die Passnut 43 vorgesehen. Durch diese Ausführungsform, bei der entsprechende V-Nuten längs der Zylinderfläche des Isolierkörpers 40, beispielsweise eines Keramikstifts, vorgesehen sind und einer entsprechenden Metallisierung einer Flanke der V-Nut ist es möglich, beispielsweise bei einer Höhe der Flanke von 45 µm bei einem Durchmesser des Isolierkörpers von 10 mm etwa 300 Leiterbahnen in einem entsprechenden Stift bzw. Isolierkörper 40 vorzusehen.

Außerdem ist in Fig. 13 dargestellt, dass die Nut 43 auch eine Leitschicht 44 aufweist, mittels der auch eine Kontaktierung vorgenommen werden kann. Beispielsweise kann die Masse von dem hermetisch dichten Raum nach außerhalb des hermetisch dichten Raums bzw. anders herum durchgeleitet werden.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein.

### Bezugszeichenliste

- 1: Videoendoskop
- 2: Systemrohr
- 3: eingelötetes Fenster
- 4: Objektiv
- 5: Videokamera
- 6: elektrische Leitung
- 7: proximale Öffnung
- 8: Vergussmasse
- 9: Durchführungsleiter
- 9^{I}: Durchführungsleiter
- 9^{II}, 9^{III}, 9^{IV}, 9^{V}, 9^{VI},: Durchführungsleiter
- 9^{VI}, 9^{VII}, 9^{VIII}: Durchführungsleiter
- 10: flexible Leiterplatte
- 11: flexible Leiterplatte
- 12: flexible Leiterplatte
- 13: flexible Leiterplatte
- 14: Isolationsschicht
- 15: Metallschicht
- 16: Wandung
- 17: hermetisches Lot
- 18: Lot
- 19: hermetischer Raum
- 20: nicht hermetischer Raum
- 21: Substrat
- 22: Zwischenschicht
- 23: Löt- und Abschirmungsschicht
- 24: Isolationsschicht
- 25: Isolationsschicht
- 26: Isolationsschicht
- 27: Isolationsschicht
- 28: erste Halbschale
- 29: zweite Halbschale
- 30: Stoßstelle
- 31: Stoßstelle
- 32: hermetisches Lot
- 39, 39^{I}, 39^{II}, 39^{III}, 39^{IV}, 39^{V}, 39^{VI}, 39^{VII}, 39^{VIII}: Durchführungsleiter
- 40: Isolierkörper
- 41, 41': Nut
- 42: Glasverguss
- 43: Passnut
- 44: Leitschicht
- 52: Rohr
- 100: flexible Leiterplatte

## Patentansprüche

1. Chirurgisches Instrument (1) mit einem hermetischen Raum (19), wobei wenigstens eine elektrische Verbindung (9-9^{VIII}) von dem hermetischen Raum (19) nach außerhalb des hermetischen Raums (19) zu einem Außenraum (20) vorgesehen ist, **dadurch gekennzeichnet, dass** die wenigstens eine elektrische Verbindung (9-9^{VIII}) auf einem elektrisch isolierenden Substrat (21, 100) aufgebracht ist, wobei wenigstens auf der elektrischen Verbindung (9-9^{VIII}) eine erste Isolationsschicht (14, 25) aufgebracht ist und auf der ersten Isolationsschicht (14, 25) eine weitere Schicht (15, 23) aufgebracht ist, die für eine hermetische Verbindung (17, 32) geeignet ist und die weiterhin mit einer Wandung (16, 28, 29) des chirurgischen Instruments (1) hermetisch verbunden ist.

2. Chirurgisches Instrument (1) nach Anspruch 1, **dadurch gekennzeichnet, dass** die hermetische Verbindung eine Lötung (17, 32) ist.

3. Chirurgisches Instrument (1) nach Anspruch 1 oder 2, **dadurch gekennzeichnet, dass** der hermetische Raum (19) in einem Rohr (2), insbesondere eines Endo-, insbesondere eines Laparoskops, vorgesehen ist.

4. Chirurgisches Instrument (1) nach einem der Ansprüche 1 bis 3, **dadurch gekennzeichnet, dass** das elektrisch isolierende Substrat (21, 100) eine Wärmeleitfähigkeit von mehr als 1W/mK, insbesondere größer 200 W/mK, aufweist.

5. Chirurgisches Instrument (1) nach einem der Ansprüche 1 bis 4, **dadurch gekennzeichnet, dass** das elektrisch isolierende Substrat (21, 100) wenigstens ein Teil einer flexiblen Leiterplatte (100) ist, wobei insbesondere die wenigstens eine elektrische Verbindung (9-9^{VIII}) wenigstens ein Leiter der Leiterplatte (100) ist.

6. Chirurgisches Instrument (1) nach einem der Ansprüche 1 bis 5, **dadurch gekennzeichnet, dass** mehrere elektrische Verbindungen (9-9^{VIII}) vorgesehen sind, die, insbesondere symmetrisch, um das Substrat (21, 100) angeordnet sind.

7. Chirurgisches Instrument (1) nach einem der Ansprüche 3 bis 6, **dadurch gekennzeichnet, dass** sich von dem Rohr (2) im Bereich des elektrisch isolierenden Substrats (21, 100) eine Wandung (16, 28, 29), insbesondere radial, nach innen erstreckt, wobei die Wandung (16, 28, 29), insbesondere radial, das elektrisch isolierende Substrat (21, 100) umgibt und mit der für die hermetische Verbindung geeigneten Schicht (15, 23) umlaufend hermetisch verbunden ist.

8. Chirurgisches Instrument (1) nach Anspruch 7, **dadurch gekennzeichnet, dass** die Wandung (16, 28, 29) wenigstens zwei Schalenelemente (28, 29) umfasst, die insbesondere miteinander hermetisch gefügt sind.

9. Chirurgisches Instrument (1) mit einem hermetischen Raum (19), wobei eine Mehrzahl von elektrischen Verbindungen (9-9^{VIII}; 39-39^{VIII}) von dem hermetischen Raum (19) nach außerhalb des hermetischen Raums (19) zu einem Außenraum (20) vorgesehen ist, insbesondere nach einem der Ansprüche 1 bis 8, **dadurch gekennzeichnet, dass** die Mehrzahl von elektrischen Verbindungen (9-9^{VIII}; 39-39^{VIII}) auf einer Oberfläche eines elektrisch isolierendem Körpers (40) aufgebracht sind, wobei der Körper (40) eine Längserstreckung von dem hermetischen Raum (19) nach außerhalb des hermetischen Raums (19) aufweist, wobei der Körper (40) hermetisch mit einem Rohr (2, 52) verbunden ist, wobei die Oberfläche des Körpers (40) mit der Mehrzahl von elektrischen Verbindungen (9-9^{VIII}; 39-39^{VIII}) bedruckt ist und/oder die elektrischen Verbindungen (9-9^{VIII}; 39-39^{VIII}) in Form einer Metallisierung auf die Oberfläche des Körpers (40) aufgebracht sind.

10. Chirurgisches Instrument (1) nach Anspruch 9, **dadurch gekennzeichnet, dass** der Körper (40) längsaxial sich erstreckende Nuten (41, 42') aufweist, wobei wenigstens ein Teil der Mehrzahl der elektrischen Verbindungen (39-39^{VIII}) in den Nuten (41, 41') angeordnet sind.

11. Chirurgisches Instrument (1) nach Anspruch 10, **dadurch gekennzeichnet, dass** die Nuten (41, 41') einen rechteckigen oder V-förmigen Querschnitt aufweisen.

12. Chirurgisches Instrument (1) nach Anspruch 10 oder 11, **dadurch gekennzeichnet, dass** im Fall der V-förmigen Nuten (41') jeweils eine elektrische Verbindung (39-39^{VIII}) auf eine Flanke der jeweiligen Nut (41') aufgebracht ist.

13. Chirurgisches Instrument (1) nach Anspruch 10 oder 11, **dadurch gekennzeichnet, dass** ein Teil der Mehrzahl der elektrischen Verbindungen (9-9^{VIII}) außerhalb der Nuten (41, 41') vorgesehen ist.

14. Chirurgisches Instrument nach einem der Ansprüche 9 bis 13, **dadurch gekennzeichnet, dass** das Rohr (52) das Außenrohr (2) des chirurgischen Instruments (1) ist oder mit dem Außenrohr (2) des chirurgischen Instruments (1) hermetisch abgeschlossen ist.

## Claims

1. A surgical instrument (1) having a hermetic chamber (19), wherein at least one electrical connection (9-9^{VIII}) is provided from the hermetic chamber (19) to outside the hermetic chamber (19) to an outer chamber (20), **characterized in that** the at least one electrical connection (9-9^{VIII}) is applied to an electrically insulating substrate (21, 100), wherein a first insulation layer (14, 25) is applied at least to the electrical connection (9-9^{VIII}), and another layer (15, 23) is applied to the first insulation layer (14, 25) that is suitable for a hermetic connection (17, 32) and that furthermore is hermetically connected to a wall (16, 28, 29) of the surgical instrument (1).

2. The surgical instrument (1) according to claim 1, **characterized in that** the hermetic connection is a soldering (17, 32).

3. The surgical instrument (1) according to claim 1 or 2, **characterized in that** the hermetic chamber (19) is provided in a tube (2), in particular an endoscope, in particular a laparoscope.

4. The surgical instrument (1) according to one of claims 1 to 3, **characterized in that** the electrically insulating substrate (21, 100) has a thermal conductivity greater than 1W/mK, in particular greater than 200 W/mK.

5. The surgical instrument (1) according to one of claims 1 to 4, **characterized in that** the electrically insulating substrate (21, 100) is at least one part of a flexible printed circuit board (100), wherein in particular the at least one electrical connection (9-9^{VIII}) is at least one trace of the printed circuit board (100).

6. The surgical instrument (1) according to one of claims 1 to 5, **characterized in that** a plurality of electrical connections (9-9^{VIII}) are provided that are arranged in particular symmetrically around the substrate (21, 100).

7. The surgical instrument (1) according to one of claims 3 to 6, **characterized in that** a wall (16, 28, 29) extends inwardly, in particular radially, from the tube (2) in the region of the electrically insulating substrate (21, 100), wherein the wall (16, 28, 29) surrounds the electrically insulating substrate (21, 100), in particular radially, and is hermetically connected peripherally to the layer (15, 23) suitable for the hermetic connection.

8. The surgical instrument (1) according to claim 7, **characterized in that** the wall (16, 28, 29) comprises at least two shell elements (28, 29) that are in particular hermetically joined to each other.

9. The surgical instrument (1) having a hermetic chamber (19), wherein a plurality of electrical connections (9-9^{VIII}; 39-39^{VIII}) is provided from the hermetic chamber (19) to the outside of the hermetic chamber (19) to an outer chamber (20), in particular according to one of claims 1 to 8, **characterized in that** the plurality of electrical connections (9-9^{VIII}; 39-39^{VIII}) is applied to a surface of an electrically insulating body (40), wherein the body (40) has a longitudinal extension from the hermetic chamber (19) to the outside the hermetic chamber (19), wherein the body (40) is hermetically connected to a tube (2, 52), wherein the surface of the body (40) is printed with the plurality of electrical connections (9-9^{VIII}; 39-39^{VIII}), and/or the electrical connections (9-9^{VIII}; 39-39^{VIII}) are applied to the surface of the body (40) in the form of metalization.

10. The surgical instrument (1) according to claim 9, **characterized in that** the body (40) has grooves (41, 42') that extend along the longitudinal axis, wherein at least part of the plurality of the electrical connections (39-39^{VIII}) is arranged in the grooves (41, 41').

11. The surgical instrument (1) according to claim 10, **characterized in that** the grooves (41,41') have a rectangular or V-shaped cross-section.

12. The surgical instrument (1) according to claim 10 or 11, **characterized in that** in the case of V-shaped grooves (41'), an electrical connection (39-39^{VIII}) is applied to one side of the respective groove (41').

13. The surgical instrument (1) according to one of claims 10 or 11, **characterized in that** part of the plurality of the electrical connections (9-9^{VIII}) is provided outside the grooves (41, 41').

14. The surgical instrument according to one of claims 9 to 13, **characterized in that** the tube (52) is the outer tube (2) of the surgical instrument (1), or is hermetically sealed to the outer tube (2) of the surgical instrument (1).

## Revendications

1. Un instrument chirurgical (1) comprenant un espace hermétique (19), dans lequel au moins une connexion électrique (9-9^{VIII}) est prévue depuis l'espace hermétique (19) vers l'extérieur de l'espace hermétique (19), vers un espace extérieur (20), **caractérisé en ce que** ladite au moins une connexion électrique (9-9^{VIII}) est appliquée sur un substrat électriquement isolant (21, 100), dans lequel au moins une première couche isolante (14, 25) est appliquée sur la connexion électrique (9-9^{VIII}) et une autre couche (15, 23) est appliquée sur ladite première couche isolante (14, 25), ce qui est adapté pour une liaison hermétique (17, 32), laquelle est en outre hermétiquement jointe à une paroi (16, 28, 29) de l'instrument chirurgical (1).

2. Instrument chirurgical (1) selon la revendication 1, **caractérisé en ce que** la liaison étanche est une brasure (17, 32).

3. Instrument chirurgical (1) selon la revendication 1 ou la revendication 2, **caractérisé en ce que** l'espace hermétique (19) est prévu dans un tube (2), en particulier dans un endoscope, en particulier un laparoscope.

4. Instrument chirurgical (1) selon l'une des revendications 1 à 3, **caractérisé en ce que** le substrat (21, 100) électriquement isolant présente une conductivité thermique supérieure à 1W/mK, en particulier supérieure à 200W/mK.

5. Instrument chirurgical (1) selon l'une des revendications 1 à 4, **caractérisé en ce que** le substrat électriquement isolant (21, 100) est au moins une partie d'une carte de circuit imprimé (100) flexible, dans lequel, en particulier, ladite au moins une connexion électrique (9-9^{VIII}) est au moins un conducteur de la carte de circuit imprimé (100).

6. Instrument chirurgical (1) selon l'une des revendications 1 à 5, **caractérisé en ce qu'**une pluralité de connexions électriques (9-9^{VIII}) est prévue, lesquelles sont disposée sur le substrat (21, 100), en particulier de façon symétrique.

7. Instrument chirurgical (1) selon l'une des revendications 3 à 6, **caractérisé en ce qu'**à partir du tube (2), dans la zone du substrat (21, 100) électriquement isolant, une paroi (16, 28, 29) s'étend vers l'intérieur, en particulier radialement, dans lequel la paroi (16, 28, 29), en particulier radiale, entoure le substrat (21, 100) électriquement isolant et est reliée hermétiquement de manière circonférentielle avec la couche de liaison hermétique (15, 23) adaptée.

8. Instrument chirurgical (1) selon la revendication 7, **caractérisé en ce que** la paroi (16, 28, 29) comprend au moins deux éléments de coque (28, 29), qui sont, en particulier, joints à elle hermétiquement.

9. Instrument chirurgical (1) avec un espace hermétique (19), dans lequel une pluralité de connexions électriques (9-9^{VIII}; 39-39^{VIII}) est prévue depuis l'espace hermétique (19) vers l'extérieur de l'espace hermétique (19) vers un espace (20), en particulier selon l'une des revendications 1 à 8, **caractérisé en ce que** la pluralité de connexions électriques (9-9^{VIII}; 39-39^{VIII}) est appliquée sur une surface d'un corps électriquement isolant (40), dans lequel le corps (40) présente une extension longitudinale allant de l'espace hermétique (19) vers l'extérieur de l'espace hermétique (19), ledit corps (40) étant hermétiquement relié à un tube (2, 52), dans lequel la pluralité de connexions électriques (9-9^{VIII}; 39-39^{VIII}) est imprimée sur la surface du corps (40) et / ou les connexions électriques (9-9^{VIII}; 39-39^{VIII}) sont appliquées sous la forme d'une métallisation sur la surface du corps (40).

10. Instrument chirurgical (1) selon la revendication 9, **caractérisé en ce que** le corps (40) comprend des rainures axiales (41, 42') s'étendant longitudinalement, dans lequel au moins une partie de la pluralité de connexions électriques (39-39^{VIII}) est disposée dans les rainures (41,41').

11. Instrument chirurgical (1) selon la revendication 10, **caractérisé en ce que** les rainures (41, 41') ont une section transversale rectangulaire ou en forme de V.

12. Instrument chirurgical (1) selon la revendication 10 ou la revendication 11, **caractérisé en ce que**, dans le cas de rainures (41') en forme de V, chacune présente une connexion électrique (39-39^{VIII}) sur un flanc de la rainure respective (41').

13. Instrument chirurgical (1) selon la revendication 10 ou la revendication 11, **caractérisé en ce qu'**une partie de la pluralité de connexions électriques (39-39^{VIII}) est prévue à l'extérieur des fentes (41,41').

14. Instrument chirurgical selon l'une quelconque des revendications 9 à 13, **caractérisé en ce que** le tube (52) est le tube extérieur (2) de l'instrument chirurgical (1) ou est scellé hermétiquement au tube extérieur (2) de l'instrument chirurgical (1).
